# EUROPEAN PATENT SPECIFICATION

(11) **EP 0 769 294 B1**
(45) Date of publication and mention of the grant of the patent: **12.12.2001**
(21) Application number: 96307457.0
(22) Date of filing: 14.10.1996
(51) Int. Cl.: A61K 9/14, A61K 9/16, A61K 47/10

(54) **Taste-masked aqueous solutions comprising ibuprofen and menthol**
Geschmacksmaskierte wässrige Lösungen enthaltend Ibuprofen und Menthol
Solutions aqueuses masquant le goût contenant de l'ibuprofen et du menthol

(30) Priority: 17.10.1995 GB 9521251; 18.10.1995 GB 9521367
(43) Date of publication of application: 23.04.1997
(73) Proprietor: Reckitt Benckiser Healthcare (UK) Limited, Slough, Berkshire SL1 3UH (GB)
(72) Inventor: Allison, Robert, North Ferriby, North Humberside HU14 3RG (GB); McFarthing, Kevin Gerard, Near Driffield, North Humberside (GB)
(74) Representative: Sanderson, Nigel Paul

(56) References cited:
- EP-A- 0 429 856
- WO-A-94/10994
- US-A- 4 918 103
- US-A- 5 190 981
- US-A- 5 206 262

## Description

This invention relates to pharmaceutical compositions containing ibuprofen having improved taste characteristics in aqueous solution. More specifically the compositions have reduced bitter taste and pleasant flavour characteristics.

Ibuprofen (2-isobutylphenyl-propionic acid) is well-known as a valuable pharmaceutical agent, endowed with analgesic and antipyretic properties; its use is now broadly accepted, and in appropriate doses it is classified among the safe pharmaceutical agents not requiring a prescription.

Ibuprofen is known to have poor organoleptic properties (including a bitter taste and causing a burning sensation in the throat) and numerous ways of overcoming these properties have been suggested. A number of these suggestions involve bypassing the poor organoleptic properties by the use of swallowable tablets or capsules, thus reducing or eliminating contact between the bitter agents and the taste receptors in the mouth and throat. Many people, however, do not find such compositions pleasant to take; and for large proportions of the population (e.g. young children or elderly people) large solid dosage forms are medically contraindicated.

To overcome the above problems pharmaceutical compositions may be administered as a drink, i.e. as aqueous solutions. Compositions suitable to be administered as drinks may be provided as ready to use liquids, or more conveniently, in dry form (e.g. as tablets, powders or granules) to be dissolved in water by the consumer. Where the compositions are in dry form they may be added to water of any temperature before consumption. Preferably they are added to hot water because of the soothing/warming effect perceived by the consumer.

It is well known that drinks containing ibuprofen are generally unpleasant to take, because of the poor organoleptic properties of this compound as described previously. One way of partially reducing these problems is to use a soluble salt of the ibuprofen and so reduce the lingering burning effect in the throat caused by undissolved particles. The soluble salt may be incorporated in the compositions directly, formed during their manufacture or even formed when the compositions are dissolved. Of course in any case the pH of the drink must be high enough to prevent the soluble salt being converted back to the insoluble acid form.

Compositions containing soluble salts of ibuprofen are also known to have poor taste characteristics but these can be at least partly reduced by the use of appropriate flavouring agents, for example, lemon flavours.

We have now found that a drawback of using soluble ibuprofen salts in drink compositions is that the perceived characteristics of many flavouring agents tend to be adversely affected. Specifically the lack of acidity and hence sharpness means that many flavours are perceived as flat and of inferior quality. Thus the flavours are less effective in covering the taste of the ibuprofen and compositions having poor acceptability to the consumer result.

We have now surprisingly found that many flavours in such previously described drinks may be enhanced by the inclusion of a menthol flavour.
There is therefore provided a pharmaceutical composition which when dissolved in water provides an aqueous solution comprising
a) 200 to 800 mg of ibuprofen in the form of the potassium or sodium salt, the acid form or a mixture thereof;
b) from 1 mg to 2000 mg of a flavouring agent to provide a pleasant flavour; and
c) 1 to 50 mg menthol.

The composition of the invention preferably comprises 300 to 600mg of the ibuprofen. The composition of the invention especially comprises 350 to 500 mg of the ibuprofen.

The compositions of the invention have improved taste characteristics in aqueous solution.

There is further provided an aqueous solution comprising a pharmaceutical composition as hereindefined.

The compositions of the invention may be dissolved in any suitable volume of water to produce a palatable drink. Preferably, the compositions of the invention may be dissolved in from 50 to 500 ml of water, more preferably 100 to 400 ml and most preferably about 150 to 250 ml of water.

Preferably, the compositions of the invention will be dissolved in hot, but not boiling, water; more preferably the water will be from 50 to 90°C.

The compositions of the invention may comprise ibuprofen, preferably in the form of the sodium or potassium salt. Where the compositions of the invention comprise all or part of the ibuprofen in the acid form they will also comprise at least a sufficient amount of a soluble source of sodium or potassium ions to convert substantially all of the ibuprofen into a soluble salt in solution.

The compositions of the invention will most preferably contain at least a sufficient amount of a soluble source of sodium or potassium ions to neutralise substantially all of the acid components of the composition in aqueous solution.

The soluble source of sodium or potassium ions will preferably comprise sodium carbonate, potassium carbonate, sodium bicarbonate or potassium bicarbonate or a mixture of two or more such salts.

It is to be understood that where the compositions of the invention comprise a soluble salt of ibuprofen, this salt may be produced from the acid form during manufacture of the composition, by reaction of the ibuprofen and at least part of the soluble source of potassium or sodium ions.

Where the compositions of the invention initially comprise ibuprofen before its conversion to a soluble salt thereof they will preferably further comprise from 500 to 5000 mg of a soluble source of sodium or potassium ions, more preferably 600 to 3000 mg, most preferably 650 to 2500 mg.

Where the compositions of the invention initially comprise ibuprofen in the form of a soluble salt they will preferably further comprise from 0 to 1000 mg of a soluble source of sodium or potassium ions, more preferably 100 to 500 mg.

The flavouring agent is preferably a fruit flavour. More preferably the flavouring agent is selected from lemon, berry, tutti frutti, pineapple or orange flavours. Most preferably the flavouring agent is a lemon flavour.

Any pharmaceutically acceptable grades of flavouring agents may be used, for example Berries of the Forest (502.477/AP05.51, supplied by Firmenich); tutti frutti (50.377/TP05.51, supplied by Firmenich); pineapple (2M81210, supplied by Quest) or orange (51.941/AP0551, supplied by Firmenich). The flavouring agents may include natural extracts (e.g. roller dried lemon), synthetic copies of natural extracts (i.e. nature-identical flavours), completely synthetic flavours or mixtures thereof.

Preferred lemon flavouring agents include all those which comply with EC Flavourings Directive 88/388/EEC; examples of which include Lemon Tetrarome, Lemon 501.050 AP0551, Lemon 501.051 TP0551, Lemon 501.120 AP0551, Lemon 501.121 AP0551 (available from Firmenich); Lemon 630256E and Lemon 611424E (available from Tastemaker); Lemon 8476 (available from SBI); Lemon Green H.1814, Lemon E.6588 and Lemon H.V. E.10071 (available from Mane Ltd); and Lemon, Lemon BK, Lemon J1309, Lemon 5374, Lemon Celebrity 5551, Lemon 5787, Lemon Strong H7297, Lemon BK H9917, Lemon H9918 (available from BBA). It will be understood that commercially available lemon flavouring agents vary immensely in their relative flavour strengths but selection of appropriate concentrations may be easily accomplished by simple experimentation.

Preferably the compositions of the invention will comprise from 5 to 1000 mg of flavouring agents, more preferably from 10 to 750 mg and most preferably from 20 to 400 mg. It is to be understood that the weights of flavouring agents listed refer to the active flavouring agents alone, not including any carriers which may be incorporated therein.

The compositions of the invention may comprise any pharmaceutically acceptable source of menthol for example menthol BP or menthol USP. These include concentrated menthol or menthol flavour agents comprising menthol plus carriers.

Preferred forms of menthol include menthol BP and encapsulated or crystallised forms thereof.

Preferably the compositions of the invention comprise from 2 to 30 mg menthol, more preferably 5 to 20 mg, most preferably about 10 mg. It is to be understood that the weights of menthol listed refer to the amount of menthol alone, not including any carriers which may be incorporated therein.

The compositions of the invention will preferably further comprise sweetening agents. Any pharmaceutically acceptable sweetening agents may be used, in suitable amounts that may be determined by simple experiment. Preferred sweetening agents include saccharin (or the sodium salt thereof) or aspartame or mixtures thereof. Preferred amounts of saccharin and/or aspartame are 25 mg to 400 mg, most preferably about 100 mg of each.

The compositions of the invention may further comprise additional active agents depending upon the conditions they are to be used to treat. Any pharmaceutically acceptable active agents may be included in the compositions of the invention as long as they are stable in such compositions and do not adversely affect the flavour thereof.

A preferred extra active agent is pseudoephedrine hydrochloride, preferably 15 mg to 250 mg, more preferably 30 mg to 150 mg, most preferably about 60 mg.

The compositions of the invention may be provided as ready to drink liquids, or, preferably, in dry form suitable to be added to water before consumption. In dry form the compositions of the invention will preferably be in the form of soluble tablets or capsules or, most preferably, in the form of powders or granules.

Compositions of the present invention in the form of powders or granules are preferably provided in unit dosage form, for example filled into capsules, or most preferably, in sachets. However it is to be understood that they may also be provided in bulk form, such that the consumer may measure out the dose to be added to water, for example by using a standard sized spoon, or a measuring scoop provided with the bulk pack.

Further it is to be understood that where the compositions of the invention are in unit dosage form (for example as tablets, or powders or granules filled into sachets) then more than one such unit dosage form may be dissolved in the same volume of water, to produce a drink containing a composition according to the invention.

Compositions of the invention may comprise further conventional excipients as necessary, depending upon the dosage form.

In liquid form the compositions of the invention may suitably comprise, colouring agents, preservatives etc.

In dry form the compositions of the invention may further comprise effervescence producing components. Any pharmaceutically acceptable conventional effervescence producing components may be included in the compositions of the inventions.

However, it will be understood that any acid ingredients used must be complemented by the inclusion of at least a neutralising amount of a base component to prevent the ibuprofen from being converted to the acid form in solution.

A preferred effervescence producing acid component is citric acid, preferably 25 mg to 400 mg, more preferably 50 mg to 200 mg, most preferably about 100 mg. The required neutralising coeffervescent material may be provided as, for example, an excess of sodium or potassium bicarbonate.

The compositions of the invention may be manufactured by any suitable means, depending upon the dosage form.

In liquid form the compositions of the invention may be produced for example by dissolving all of the ingredients in water.

In the form of powders the compositions of the invention may for example be produced by mixing all of the ingredients and blending. The resulting powders may be filled into bulk containers, or divided into unit dosage forms in sachets or capsules or compressed into soluble tablets.

In granular form the compositions of the invention may be produced by combining all of the ingredients and granulating by wet or dry granulation; or they may, preferably, be produced as mixtures of two or more granule types, or a mixture of granules and powders, each containing different parts of the total formulation.

Where wet granulation is used to prepare the granules they may be dried by any conventional drying technique.

Granules, or mixtures of granules and powders, may be filled into bulk containers or, preferably, divided into unit dosage forms in sachets or capsules, or compressed into soluble tablets.

Compositions of the invention in the form of soluble tablets may be produced by direct compression of a mixture of all of the ingredients; or some or all of the ingredients may be granulated before mixing and compression.
The invention will now be illustrated by the following Examples.

### Example 1

| Ibuprofen / Pseudoephedrine Composition. | |
|---|---|
| Ibuprofen | 4 kg |
| Pseudoephedrine hydrochloride | 600 g |
| Sodium bicarbonate | 10 kg |
| Sodium citrate | 3.8 kg |
| Sodium carbonate | 3.05 kg |
| Citric acid anhydrous | 1 kg |
| Saccharin sodium | 1 kg |
| Sodium chloride | 400 kg |
| Docusate sodium | 30 kg |
| Riboflavin sodium phosphate | 20 kg |
| Roller dried lemon 100 % | 1.64 kg |
| Lemon flavour (JR4378, BBA) | 2 kg |
| Menthol flavour BP (20 % menthol, 80% carrier) | 500 g |

1) The ibuprofen, sodium bicarbonate and sodium carbonate were sieved and blended together. 10 g of the riboflavin was added as an aqueous solution and the mixture was granulated using deionised water as the granulating fluid. The granules were dried and cooled in a fluid bed drier and reseived to break up large aggregates.
2) The trisodium citrate, citric acid, sodium saccharin and sodium chloride were mixed and blended and 10 g of the riboflavin, plus the docusate sodium, were added as an aqueous solution. The mixture was granulated using deionised water as the granulation fluid. The resultant granules were dried and cooled in a fluid bed drier and reseived to break up large aggregates.
3) The granules from steps 1) and 2) were blended together with the pseudoephedrine hydrochloride, lemon flavour, roller dried lemon and menthol flavour.
4) The resultant mixed granules were filled into sachets, with an average fill weight of 2804 mg.

When the contents of one of the sachets prepared in step 4) are added to 200 ml of hot water and stirred well a drink containing the equivalent of 400 mg ibuprofen and 60 mg pseudoephedrine hydrochloride results. The drink has a pleasant lemon flavour. The flavour is improved compared to the same formulation without menthol.

### Example 2

Example 1 was repeated using an alternative grade of lemon flavour (XJ5358, supplied by BBA).

This composition has an improved lemon flavour when dissolved in hot water and compared to the same formulation without menthol.

### Example 3

Example 1 was repeated replacing the lemon flavour and the dried lemon by 2 kg of Berries of the Forest flavour (502.477/AP05.51, supplied by Firmenich). The sachet fill weight was 2640 mg.

This composition has an improved flavour when dissolved in hot water and compared to the same formulation without menthol.

### Example 4

Example 1 was repeated replacing the lemon flavour and the dried lemon by 2 kg of tutti frutti flavour (50. 377/TP05.51, supplied by Firmenich). The sachet fill weight was 2640 mg.

This composition has an improved flavour when dissolved in hot water and compared to the same formulation without menthol.

### Example 5

Example 1 was repeated replacing the lemon flavour and the dried lemon by 2kg of Pineapple flavour (2M81210, supplied by Quest). The sachet fill weight was 2640 mg.

This composition has an improved flavour when dissolved in hot water and compared to the same formulation without menthol.

### Example 6

Example 1 was repeated replacing the lemon flavour and the dried lemon by 2kg orange flavour (51.941/AP0551, supplied by Roche). The sachet fill weight was 2640 mg.

This composition has an improved flavour when dissolved in hot water and compared to the same formulation without menthol.

## Claims

1. A pharmaceutical composition which when dissolved in water provides an aqueous solution comprising
a) 200 to 800mg of ibuprofen in the form of the potassium or sodium salt, the acid form or a mixture thereof;
b) from 1mg to 2000mg of a fruit flavouring agent to provide a pleasant flavour and;
c) 1 to 50mg menthol.

2. A pharmaceutical composition as claimed in claim 1 which comprises 300 to 600mg of the ibuprofen.

3. A pharmaceutical composition as claimed in claim 2 which comprises 350 to 500mg of the ibuprofen.

4. A pharmaceutical composition as claimed in any one of claims 1 to 3 wherein the flavouring agent is selected from lemon, berry, tutti frutti, pineapple and orange flavours.

5. A pharmaceutical composition as claimed in claim 4 wherein the flavouring agent is a lemon flavour.

6. A pharmaceutical composition as claimed in any previous claim comprising from 2 to 30mg menthol.

7. A pharmaceutical composition as claimed in any previous claim further comprising pseudoephedrine hydrochloride.

8. A pharmaceutical composition as claimed in any previous claim in unit dosage form.

9. A pharmaceutical composition as claimed in claim 8 in the form of powders or granules filled into sachets.

10. An aqueous solution comprising a pharmaceutical composition as defined in any one of the preceding claims.

## Patentansprüche

1. Pharmazeutische Zusammensetzung, die in Wasser gelöst eine wässrige Lösung bereitstellt, umfassend
a) 200 mg bis 800 mg Ibuprofen in Form das Kalium- oder Natriumsalzes, der Säureform oder eines Gemisches davon,
b) von 1 mg bis 2000 mg eines Fruchtaromastoffes, um einen angenehmen Geschmack zu erzeugen,
c) 1 mg bis 50 mg Menthol.

2. Pharmazeutische Zusammensetzung nach Anspruch 1, die 300 mg bis 600 mg Ibuprofen umfasst.

3. Pharmazeutische Zusammensetzung nach Anspruch 2, die 350 mg bis 500 mg Ibuprofen umfasst.

4. Pharmazeutische Zusammensetzung nach einem der Ansprüche 1 bis 3, wobei der Aromastoff ausgewählt ist aus Zitrone, Beeren, Vielfrucht, Ananas und Orange.

5. Pharmazeutische Zusammensetzung nach Anspruch 4, wobei der Aromastoff ein Zitronen-Aromastoff ist.

6. Pharmazeutische Zusammensetzung nach einem der vorhergehenden Ansprüche, die von 2 mg bis 30 mg Menthol umfasst.

7. Pharmazeutische Zusammensetzung nach einem der vorhergehenden Ansprüche, die außerdem Pseudoephedrin Hydrochlorid umfasst.

8. Pharmazeutische Zusammensetzung nach einem der vorhergehenden Ansprüche in Form von Einzeldosiermengen.

9. Pharmazeutische Zusammensetzung nach Anspruch 8 in Form von in Beuteln abgefüllten Pulvern oder Granulaten.

10. Wässrige Lösung, umfassend eine pharmazeutische Zusammensetzung, wie in jedem der vorangehenden Ansprüche definiert.

## Revendications

1. Composition pharmaceutique qui, une fois dissoute dans de l'eau, donne une solution aqueuse comprenant
a) 200 à 800 mg d'ibuprofène sous forme du sel de potassium ou de sodium, sous la forme acide ou sous forme d'un de leurs mélanges ;
b) 1 mg à 2000 mg d'un agent aromatisant aux fruits destiné à conférer une saveur agréable ; et
c) 1 à 50 mg de menthol.

2. Composition pharmaceutique suivant la revendication 1, qui comprend 300 à 600 mg d'ibuprofène.

3. Composition pharmaceutique suivant la revendication 2, qui comprend 350 à 500 mg d'ibuprofène.

4. Composition pharmaceutique suivant l'une quelconque des revendications 1 à 3, dans laquelle l'agent aromatisant est choisi entre des arômes de citron, de baies, de tutti frutti, d'ananas et d'orange.

5. Composition pharmaceutique suivant la revendication 4, dans laquelle l'agent aromatisant est un arôme de citron.

6. Composition pharmaceutique suivant l'une quelconque des revendications précédentes, comprenant 2 à 30 mg de menthol.

7. Composition pharmaceutique suivant l'une quelconque des revendications précédentes, comprenant en outre du chlorhydrate de pseudo-éphédrine.

8. Composition pharmaceutique suivant l'une quelconque des revendications précédentes, sous une forme posologique unitaire.

9. Composition pharmaceutique suivant la revendication 8, sous forme de poudres ou de granules conditionnés dans des sachets.

10. Solution aqueuse comprenant une composition pharmaceutique répondant à la définition suivant l'une quelconque des revendications précédentes.
